(19) Europäisches Patentamt / European Patent Office / Office européen des brevets

(11) **EP 4 559 376 A1**

(12) **EUROPEAN PATENT APPLICATION**

(43) Date of publication:
**28.05.2025 Bulletin 2025/22**

(21) Application number: **24214418.6**

(22) Date of filing: **21.11.2024**

(51) International Patent Classification (IPC):
**A61B 1/00** (2006.01)  **A61B 1/005** (2006.01)

(52) Cooperative Patent Classification (CPC):
**A61B 1/009; A61B 1/00006**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**GE KH MA MD TN**

(30) Priority: **21.11.2023 KR 20230162122**

(71) Applicant: **Medintech Inc.**
**Seoul 03100 (KR)**

(72) Inventors:
• **KIM, Myungjoon**
**13831 Gwacheon-si, Gyeonggi-do (KR)**
• **KIM, Geono**
**02829 Seoul (KR)**
• **CHO, Seokho**
**03082 Seoul (KR)**

(74) Representative: **Isarpatent**
**Patent- und Rechtsanwälte**
**Barth Hassa Peckmann & Partner mbB**
**Friedrichstraße 31**
**80801 München (DE)**

(54) **METHOD AND APPARATUS FOR ESTIMATING POSE OF ENDOSCOPIC SCOPE**

(57) According to one embodiment of the present disclosure, there is disclosed a method of estimating a pose of an endoscopic scope that is performed by a computing device including at least one processor. The method includes estimating the pose of an endoscopic scope based on the pose information of a drive unit configured to control the movement of the scope by using a backlash model in which control parameters have been identified for backlash that occurs in the scope.

FIG. 1

**Description**

CROSS-REFERENCE TO RELATED APPLICATION

This application claims the benefit of Korean Patent Application No. 10-2023-0162122 filed on November 21, 2023, which is hereby incorporated by reference herein in its entirety.

BACKGROUND

1. Technical Field

The present disclosure relates to a method and apparatus for estimating the pose of the end of an endoscopic scope.

2. Description of the Related Art

Endoscopes collectively refer to medical devices that enable a scope to be inserted into the human body and a user to observe an organ without surgery or autopsy. Endoscopes enable a scope to be inserted into the human body, light to be radiated, and the light reflected from the surface of the inner wall of the human body to be visualized. Endoscopes are classified according to their purpose and target body part, and may be basically classified into rigid endoscopes, in which an endoscopic tube is made of metal, and flexible endoscopes, which are represented by digestive endoscopes.
A flexible endoscope contains various devices therein and is thus vulnerable to impact, and also the inside of a digestive organ into which a flexible endoscope is inserted corresponds to a considerably fragile tissue and has an irregular shape. Furthermore, the shape of the inside of the digestive organ varies depending on the patient, so that the process of inserting the endoscope may not be easy even for experienced medical professionals.
In this case, as an endoscopic procedure is performed, an endoscopic scope is inserted into a digestive organ while being twisted and turned according to the shape of the digestive organ.
In the early stage of the endoscopic procedure, an endoscopic operator may bend or move the scope to a desired angle without a large amount of force because the scope has a relatively simple shape. However, as the procedure progresses to the latter half of the procedure or when complex movement is involved during the procedure, controlling the scope while taking into consideration the characteristics of the scope that may change at any moment can cause significant inconvenience to the endoscopic operator. In addition, the nature of the endoscopic procedure in which the scope is inserted into the body requires the delicate control of the scope, and thus, there is a demand for technology for controlling an endoscopic scope by reflecting therein the physical changes that occur in an endoscope device during an endoscopic procedure.
In order to automatically control the endoscopic scope to a desired position and angle, it is first required to accurately determine the position of the endoscopic scope. However, there is a limitation on the thickness of a scope tube due to the nature of the endoscopic scope in which the endoscopic scope is inserted into the body. Therefore, it is difficult to mount various sensors for obtaining position information on an endoscopic scope, so that there is a demand for technology for estimating the exact position of a scope without mounting a separate sensor on an endoscopic scope.

SUMMARY

The present disclosure is intended to overcome the problems of the above-described conventional art, and is directed to a method and apparatus for estimating the pose of an endoscopic scope by modeling the backlash of the endoscopic scope or using an artificial neural network model.
However, objects to be achieved by the present disclosure are not limited to the object described above, and another object may be present.
According to one embodiment of the present disclosure for achieving the above-described object, there is disclosed a method of estimating a pose of an endoscopic scope that is performed by a computing device including at least one processor. The method includes estimating the pose of an endoscopic scope based on the pose information of a drive unit configured to control the movement of the scope by using a backlash model in which control parameters have been identified for backlash that occurs in the scope.
Alternatively, the backlash model may be determined according to the angle range of the scope.
Alternatively, the backlash model may be set such that the amount of backlash is constant when the angle of the scope is within a first range.
Alternatively, the backlash model may be set such that the amount of backlash increases when the angle of the scope is within a second range.

Alternatively, estimating the pose of the scope may include determining whether the scope is in a backlash state based on the backlash model and the state of the drive unit.

Alternatively, estimating the pose of the scope may include estimating that the pose of the scope is constant in a backlash state.

Alternatively, estimating the pose of the scope may include estimating the bending angle of the scope based on the angle of the motor when the scope is not in a backlash state.

According to one embodiment of the present disclosure for achieving the above-described object, there is disclosed a method of estimating the pose of an endoscopic scope, the method being performed by a computing device including at least one processor, the method including: estimating the pose of an endoscopic scope based on the pose information of a drive unit configured to control the movement of the scope by using an artificial neural network model that is trained to infer the pose of the scope by using the pose information of the drive unit as training data.

Alternatively, the pose information of the drive unit may include the pose information of a motor configured to provide power to the scope and the direction information of the motor.

According to one embodiment of the present disclosure for achieving the above-described object, there is disclosed a computing device for estimating the pose of an endoscopic scope, the computing device including: a processor including at least one core; and memory including program codes executable on the processor; wherein the processor estimates the pose of an endoscopic scope based on the pose information of a drive unit configured to control the movement of the scope by using a backlash model in which control parameters have been identified for backlash that occurs in the scope.

According to one embodiment of the present disclosure for achieving the above-described object, there is disclosed a computing device for estimating the pose of an endoscopic scope, the computing device including: a processor including at least one core; and memory including program codes executable on the processor; wherein the processor estimates the pose of an endoscopic scope based on the pose information of a drive unit configured to control the movement of the scope by using an artificial neural network model that is trained to infer the pose of the scope by using the pose information of the drive unit as training data.

According to one embodiment of the present disclosure, in a real situation where an endoscope device is operating, the risk in which an endoscopic operator performs the operation of excessively moving an endoscopic scope in a backlash section due to the fact that the scope does not move in the backlash section is reduced, and the endoscopic operator may perform a desired degree of scope manipulation precisely.

Furthermore, according to one embodiment of the present disclosure, various nonlinear features that may occur, as well as backlash, are reflected in the artificial neural network model training process, so that the pose of the scope can be estimated with higher accuracy than the method of estimating the pose of a scope using only a simple linear model.

BRIEF DESCRIPTION OF THE DRAWINGS

The above and other objects, features, and advantages of the present invention will be more clearly understood from the following detailed description taken in conjunction with the accompanying drawings, in which:

FIG. 1 is a diagram showing the configuration of endoscope device according to one embodiment of the present disclosure;

FIG. 2 is a block diagram showing a part of the configuration of an endoscope device according to one embodiment of the present disclosure;

FIG. 3 is a flowchart showing a method of estimating the pose of an endoscopic scope according to one embodiment of the present disclosure;

FIG. 4 is a graph showing a backlash model according to one embodiment of the present disclosure as an example; and

FIG. 5 is a flowchart showing a method of estimating the pose of an endoscopic scope using an artificial neural network model according to one embodiment of the present disclosure.

DETAILED DESCRIPTION

Embodiments of the present disclosure will be described in detail below with reference to the accompanying drawings so that those having ordinary skill in the art of the present disclosure (hereinafter referred to as those skilled in the art) can easily implement the present disclosure. The embodiments presented in the present disclosure are provided to enable those skilled in the art to use or practice the content of the present disclosure. Accordingly, various modifications to embodiments of the present disclosure will be apparent to those skilled in the art. That is, the present disclosure may be implemented in various different forms and is not limited to the following embodiments.

The same or similar reference numerals denote the same or similar components throughout the specification of the present disclosure. Additionally, in order to clearly describe the present disclosure, reference numerals for parts that are not related to the description of the present disclosure may be omitted in the drawings.

The term "or" used herein is intended not to mean an exclusive "or" but to mean an inclusive "or." That is, unless otherwise specified herein or the meaning is not clear from the context, the clause "X uses A or B" should be understood to mean one of the natural inclusive substitutions. For example, unless otherwise specified herein or the meaning is not clear from the context, the clause "X uses A or B" may be interpreted as any one of a case where X uses A, a case where X uses B, and a case where X uses both A and B.

The term "and/or" used herein should be understood to refer to and include all possible combinations of one or more of listed related concepts.

The terms "include" and/or "including" used herein should be understood to mean that specific features and/or components are present. However, the terms "include" and/or "including" should be understood as not excluding the presence or addition of one or more other features, one or more other components, and/or combinations thereof.

Unless otherwise specified herein or unless the context clearly indicates a singular form, a singular form should generally be construed to include "one or more."

The term "N-th (N is a natural number)" used herein can be understood as an expression used to distinguish the components of the present disclosure according to a predetermined criterion such as a functional perspective, a structural perspective, or the convenience of description. For example, in the present disclosure, components performing different functional roles may be distinguished as a first component or a second component. However, components that are substantially the same within the technical spirit of the present disclosure but should be distinguished for the convenience of description may also be distinguished as a first component or a second component.

Meanwhile, the term "module" or "unit" used herein may be understood as a term referring to an independent functional unit that processes computing resources, such as a computer-related entity, firmware, software or part thereof, hardware or part thereof, or a combination of software and hardware. In this case, the "module" or "unit" may be a unit composed of a single component, or may be a unit expressed as a combination or set of multiple components. For example, in the narrow sense, the term "module" or "unit" may refer to a hardware component or set of components of a computing device, an application program performing a specific function of software, a procedure implemented through the execution of software, a set of instructions for the execution of a program, or the like. Additionally, in the broad sense, the term "module" or "unit" may refer to a computing device itself constituting part of a system, an application running on the computing device, or the like. However, the above-described concepts are only examples, and the concept of "module" or "unit" may be defined in various manners within a range understandable to those skilled in the art based on the content of the present disclosure.

The term "model" used herein may be understood as a system implemented using mathematical concepts and language to solve a specific problem, a set of software units intended to solve a specific problem, or an abstract model for a process intended to solve a specific problem. For example, a neural network "model" may refer to an overall system implemented as a neural network that is provided with problem-solving capabilities through training. In this case, the neural network may be provided with problem-solving capabilities by optimizing parameters connecting nodes or neurons through training. The neural network "model" may include a single neural network, or a neural network set in which multiple neural networks are combined together.

The foregoing descriptions of the terms are intended to help to understand the present disclosure. Accordingly, it should be noted that unless the above-described terms are explicitly described as limiting the content of the present disclosure, the terms in the content of the present disclosure are not used in the sense of limiting the technical spirit of the present disclosure.

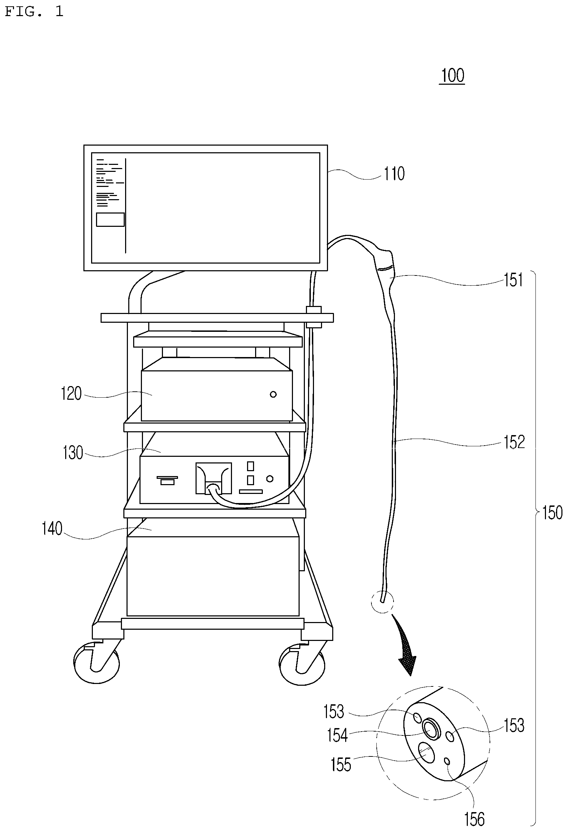

FIG. 1 is a diagram showing the configuration of endoscope device according to one embodiment of the present disclosure.

Referring to FIG. 1, an endoscope device 100 according to one embodiment of the present disclosure may be a flexible endoscope, or more specifically, a digestive endoscope. The endoscope device 100 may include a configuration capable of obtaining a medical image adapted to photograph the inside of a digestive organ and a configuration capable of, when necessary, allowing a tool to be inserted and a user to perform treatment or manipulation while viewing a medical image.

The endoscope device 100 may include an output unit 110, a control unit 120, a drive unit 130, a pump unit 140, and a scope 150, and may further include a light source unit (not shown).

The output unit 110 may include a display configured to display medical images. The output unit 110 may include a display module configured to output visualized information or implement touch screens, such as a liquid crystal display (LCD), a thin film transistor-liquid crystal display (TFT LCD), an organic light-emitting diode (OLED), a flexible display, a three-dimensional (3D) display, or the like.

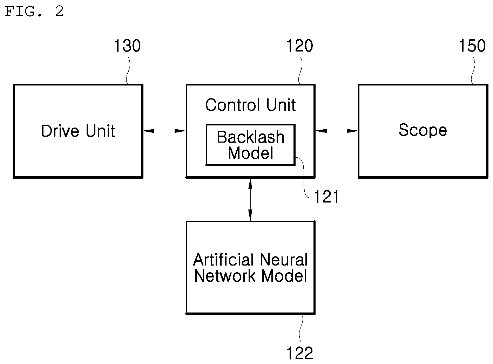

The output unit 110 may include various means for providing medical images or information about medical images. The output unit 110 may display medical images obtained by the scope 150 or medical images processed by the control unit 120. The output unit 110 may provide information through an auditory means in addition to a visual means, and may include, for example, a speaker configured to provide alarms regarding medical images audibly. Meanwhile, although the one output unit 110 is illustrated in FIG. 2, a plurality of output units 110 may be provided. In this case, an output unit 110 where a medical image obtained by the scope 150 is displayed and an output unit 110 where information processed by the control unit 120 is displayed may be separated from each other.

The control unit 120 may control the overall operation of the endoscope device 100. For example, the control unit 120 may perform the operation of taking a medical image through the scope 150, the operation of processing an obtained medical image, the control operation of performing medical operations such as the spraying of washing water, suction, or the like, a series of operations for controlling the movement of the scope 150, etc. The control unit 120 may include all types of devices capable of processing data. According to an exemplary embodiment, the control unit 120 may be a data processing device embedded in hardware that has circuits physically structured to perform the functions represented by the codes or instructions included in a program. As an example of the data processing device embedded in hardware, a processing device such as a microprocessor, a central processing unit (CPU), a processor core, a multiprocessor, an application-specific integrated circuit (ASIC), or a field programmable gate array (FPGA) may be included, but the technical spirit of the present disclosure is not limited thereto.

The endoscope device 100 of the present invention may include a computing device including a processor and memory. For example, the computing device may constitute the control unit 120 of the endoscope device 100. That is, the computing device may be configured to perform the operation of the control unit 120.

The processor according to an embodiment of the present disclosure may be understood as a constituent unit including hardware and/or software for performing computing operation. For example, the processor may read a computer program and perform data processing for machine learning. The processor may process computational processes such as the processing of input data for machine learning, the extraction of features for machine learning, and the calculation of errors based on backpropagation. The processor for performing such data processing may include a central processing unit (CPU), a general purpose graphics processing unit (GPGPU), a tensor processing unit (TPU), an application specific integrated circuit (ASIC), or a field programmable gate array (FPGA). Since the types of processor described above are only examples, the type of processor may be configured in various manners within a range understandable to those skilled in the art based on the content of the present disclosure.

The memory according to an embodiment of the present disclosure may be understood as a constituent unit including hardware and/or software for storing and managing data that is processed in the computing device. That is, the memory may store any type of data generated or determined by the processor and any type of data received by the network unit. For example, the memory may include at least one type of storage medium of a flash memory type, hard disk type, multimedia card micro type, and card type memory, random access memory (RAM), static random access memory (SRAM), read-only memory (ROM), electrically erasable programmable read-only memory (EEPROM), programmable read-only memory (PROM), magnetic memory, a magnetic disk, and an optical disk. Furthermore, the memory may include a database system that controls and manages data in a predetermined system. Since the types of memory described above are only examples, the type of memory may be configured in various manners within a range understandable to those skilled in the art based on the content of the present disclosure. The memory may be a non-transitory medium configured to store data and instructions in a tangible form, and is not a mere signal or transitory form.

The memory may structure, organize and then manage data required for the processor to perform operations, combinations of the data, and program codes executable on the processor. Furthermore, the memory may also store program codes adapted to operate the processor to generate training data.

The computing device may further include a network unit configured to transmit and receive data to and from an external computing device, another endoscope device, a hospital server, or the like.

The control unit 120 may control the movement of the scope 150 through the drive unit 130 connected to the scope 150. That is, the control unit 120 may generate control signals to be provided to the drive unit 130 in order to control the movement of the scope 150.

For example, a series of operations in which the endoscope device 100 of the present disclosure controls the scope 150 may be performed as follows. A user may input the degree or direction of curvature of the scope 150 through a manipulation portion 151. The input information is transmitted to the control unit 120, and the control unit 120 may process the input information and generate a signal to be provided to the drive unit 130. For example, the control unit 120 may calculate the position, angle, angular velocity, and/or like of a motor corresponding to the degree or direction of curvature set by the user and may provide them to the drive unit 130. The drive unit 130 may generate power based on the signal of the control unit 120 and transmit it to the scope 150. Accordingly, the scope 150 may be moved or bent in accordance with the value input by the user.

The endoscope device 100 according to the present disclosure may estimate the pose of the scope 150. In this case, the pose of the scope 150 may refer to the pose of an insertion part configured to be inserted into a digestive organ as at least a part of the scope 150. The pose of the scope 150 may include at least one of the x-axis position, y-axis position, z-axis position, roll, pitch, and yaw values of the end of the scope 150 and a bending angle measured according to preset criteria.

The endoscope device 100 may model the backlash that occurs at the end of the scope 150 to estimate the pose of the scope 150, and may estimate the pose of the scope 150 based on a backlash model.

In the present disclosure, the term "backlash" refers to a phenomenon in which the force generated from the drive unit 130 is transmitted through a wire inside the scope 150 but the movement of the end of the scope 150 does not occur. Power is generated from the drive unit 130 and provided to the wire inside the scope 150 so that an endoscopic operator can control the pose of the scope 150 to a desired degree according to the manipulation of thee endoscopic operator. In this case, there may occur the backlash in which the pose of the scope 150 does not change. Such backlash is a nonlinear element that makes the operation of controlling the scope 150 unpredictable. Accordingly, through this disclosure, the more precise and accurate control of the scope 150 may be made possible by modeling the backlash generated at the end of the scope 150 and reflecting it in the control of the scope 150.

Meanwhile, the endoscope device 100 may train an artificial neural network model to infer the pose of the scope 150 by using the pose information of the drive unit 130, controlling the movement of the scope 150, as training data. Alternatively, the endoscope device 100 may receive a trained artificial neural network model from an external computing device connected to the endoscope device 100 via a wired or wireless connection. Alternatively, the endoscope device 100 may provide training data to an external computing device, may provide input data after training has been completed, and may receive inference results.

The artificial neural network model 200 that infers the pose of the scope 150 may include at least one neural network. The neural network may include network models such as a deep neural network (DNN), a recurrent neural network (RNN), a bidirectional recurrent deep neural network (BRDNN), a multilayer perceptron (MLP), a convolutional neural network (CNN), and a transformer, but is not limited thereto.

The artificial neural network model 200 may be trained through supervised learning that uses the training data 220 as input values. Alternatively, the artificial neural network model 200 may be trained through unsupervised learning that discovers criteria for data recognition by learning the types of data required for data recognition on its own without any guidance. Alternatively, the artificial neural network model 200 may be trained through reinforcement learning that uses feedback on whether the results of data recognition according to learning are correct. Specific details for each type of learning will be described later in conjunction with FIG. 2.

Through this, when a real-use environment in which the information obtained through the endoscopic scope 150 is limited is taken into consideration, the pose of the scope 150 may be estimated using obtainable information. In addition, the different endoscopic scope manipulation style of each endoscopic operator, the irregular twisting of the scope 150, and the body structure of a patient who is the subject of an endoscopic procedure are not uniform, making it difficult to perform prediction. Therefore, according to the present disclosure, various nonlinear features that may occur, as well as backlash, are reflected in the artificial neural network model training process, so that the pose of the scope 150 can be estimated with higher accuracy than the method of estimating the pose of the scope 150 using only a simple linear model.

The drive unit 130 may provide power required for the scope 150 to be inserted into the body, bent, and moved inside the body. For example, the drive unit 130 may include the motor connected to the wire inside the scope 150 and a tension adjustment unit configured to adjust the tension of the wire.

The drive unit 130 may control the scope 150 in various directions by controlling the power of the motor. For example, the motor may be configured to include a plurality of motors corresponding to the directions in which the insertion portion 152 at the end of the scope 150 is to be bent. Alternatively, the motor may be configured to include a plurality of motors corresponding to wires inside the scope 150. More specifically, the drive unit 130 may include a first motor configured to determine the x-axis movement of the scope 150 and a second motor configured to determine the y-axis movement of the scope 150. The x-axis position, y-axis position, z-axis position, roll, pitch, and yaw values of the end of the scope 150 may be determined according to the control of the drive unit 130, but the configuration of the drive unit 130 is not limited thereto.

The tension adjustment unit may receive power from the motor and pull the wire inside the scope 150 to generate tension. Through this, the scope 150 may be bent. The tension adjustment unit 330 may adjust the tension applied to the plurality of wires 1000 inside the scope 150 so that the scope 150 can be bent according to the determined amount and direction of bending.

The pump unit 140 may include at least one of an air pump configured to inject air into the body through the scope 150, a suction pump configured to provide negative pressure or vacuum and suck air from the body through the scope 150, and a water pump configured to inject cleaning water into the body through the scope 150. Each of the pumps may include a valve configured to control the flow of fluid. The pump unit 140 may be opened and closed by the control unit 120. At least one of the suction pump, the water pump, and the air pump may be opened and closed based on a control signal of a computing device or the control of the control unit 120.

The scope 150 may include the insertion portion 152 configured to be inserted into a digestive organ and the operating portion 151 configured to control the movement of the insertion portion 152 and receive input from a user to perform various operations.

The insertion portion 152 is configured to be flexibly bent and is connected to the drive unit 130 at one end thereof, so that the degree or direction of curvature can be determined by the drive unit 130. Since medical imaging and treatment are performed at the end of the insertion portion 152, the scope 150 may include various cables and tubes that extend to the end of the insertion portion 152. A light source lens 153, an objective lens 154, a working channel 155, and an air and water channel 156 may be provided inside the scope 150. A tool for treating and managing a lesion may be inserted through the working channel 155 during an endoscopic procedure. Air may be injected and washing water may be fed through the air and water channel 156. Meanwhile, in FIG. 2, the air and water channel 156 is illustrated as a passage through which washing water is fed, but it is not limited thereto. For example, a separate water jet channel (not shown) may be provided inside the scope 150, and cleaning water may also be fed through a water jet channel.

Meanwhile, references herein to the scope 150 being bent by the control unit 120 or the drive unit 130 may refer to at least a part of the scope 150, e.g., the insertion portion 152, being bent.

The manipulation portion 151 may include a plurality of input buttons configure to provide various functions (image capture, the spray of cleaning water, etc.) to allow an endoscopic operator to control the steering of the insertion portion 152 and perform a procedure through the working channel 155 and the air and water channel 156. For example, the manipulation portion 151 may include a plurality of buttons or a joystick-type input device configured to indicate the direction of the scope 150.

The light source unit may include a light source that radiates light into the body through the endoscopic scope 150. The light source unit may include a lighting device configured to generate white light, or may include a plurality of lighting devices configured to generate rays of light having different wavelength bands. The type of light source, the intensity of light, white balance, and/or the like may be set through the light source unit. Meanwhile, the above-described setting items may also be set through the control unit 120. The light generated by the light source unit may be transmitted to the scope 150 through a path such as an optical fiber.

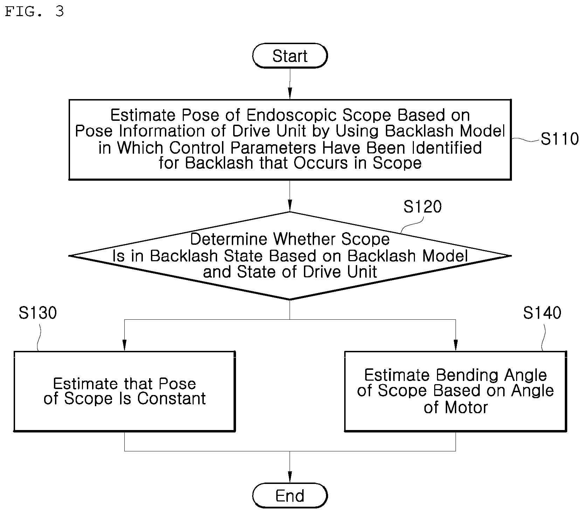

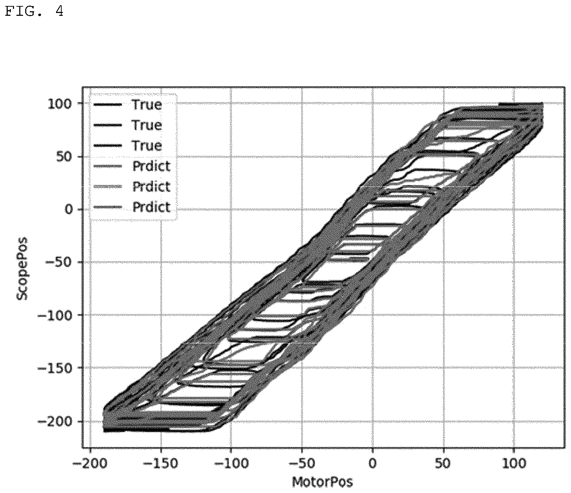

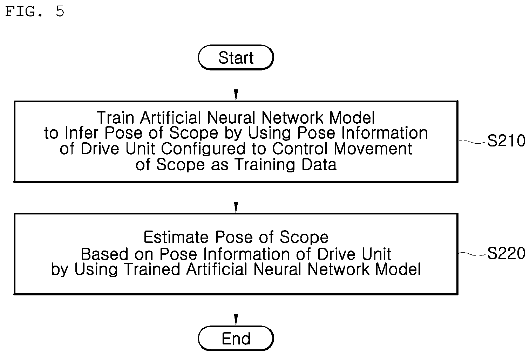

FIG. 2 is a block diagram showing a part of the configuration of an endoscope device according to one embodiment of the present disclosure, FIG. 3 is a flowchart showing a method of estimating the pose of an endoscopic scope according to one embodiment of the present disclosure, FIG. 4 is a graph showing a backlash model according to one embodiment of the present disclosure as an example, and FIG. 5 is a flowchart showing a method of estimating the pose of an endoscopic scope using an artificial neural network model according to one embodiment of the present disclosure.

Referring to FIGS. 1 to 5, the control unit 120 of the endoscope device 100 may estimate the pose of the scope 150 based on the pose information of the drive unit 130 configured to control the movement of the scope 150 by using a backlash model 121 in which control parameters have been identified for the backlash that occurs in the scope 150 in step S110.

Although the following description will be given on the assumption that the backlash model 121 is generated by the control unit 120, a modeling task for backlash may be performed by an external server connected to the endoscope device 100 via a wired and/or wireless connection.

The control unit 120 may collect the pose information of the scope 150 and the pose information of the motor in order to model the backlash.

In the graph of FIG. 4, the x axis represents the pose information of the motor and the y axis represents the pose information of the scope 150. Furthermore, the black lines represent real values, and the gray lines represent estimated values. In this case, a section in which the pose of the scope 150 does not change even when the pose of the motor changes occurs, and this section may be defined as a backlash section. In other words, a section in which the y-axis value does not change even when the x-axis value changes in the graph, i.e., a section that forms a parallel line with the x axis, may be defined as a backlash section. The backlash section may become longer as the pose of the motor moves away from 0. Accordingly, the control unit 120 may generate the backlash model 121 having a hysteresis form. However, the form of the backlash model 121 may be formed in various manners according to the type of motor, the type of scope, and components of the drive unit 130 other than the motor.

The backlash model 121 is determined according to the angle range of the scope 150, and may be implemented in different forms of formulas according to the angle range. That is, the backlash model 121 may be set such that the amount of backlash is constant when the angle of the scope 150 is within a first range and the amount of backlash increases in proportion to the increase in the angle of the scope 150 when the angle of the scope 150 is within a second range. When the angle of the scope 150 is within the second range, the backlash model 121 may be implemented in the form of an n-th order polynomial function. The degree of the polynomial function and the values of the coefficients constituting the polynomial function may be determined through regression analysis. The values of the control parameters constituting the backlash model 121 may be determined in a graph fitting process through regression analysis.

The control unit 120 may determine whether the scope 150 is in a backlash state based on the backlash model 121 in which the control parameters have been identified through the above-described process and the state of the drive unit 130 in step S120. For example, referring to the graph of FIG. 4, backlash may occur in a section where the moving direction of the motor changes. Accordingly, the control unit 120 may determine whether the scope 150 is in a backlash state based on the sign of the moving direction of the motor. For example, the control unit 120 may define a specific time section, in which the sign of the moving direction of the motor changes, as a section in a backlash state. Furthermore, the control unit 120 may determine that the scope 150 is not in a backlash state in the remaining sections excluding the section in a backlash state.

The control unit 120 may estimate that the pose of the scope 150 is constant in a backlash state in step S130. That is, in the section in a backlash state, the control unit 120 may determine that the pose of the scope 150 is the same as the pose of the scope 150 in the previous time section. In this case, the pose of the scope 150 may refer to the bending angle of the scope 150.

The control unit 120 may estimate the bending angle of the scope 150 based on the angle of the motor when the scope 150 is not in a backlash state in step S140. For example, the control unit 120 may estimate the bending angle of the scope 150 in a current time section based on the angle of the motor and the bending angle of the scope 150 in a previous time section. More specifically, the bending angle of the scope 150 may be estimated according to the following equation by using the slope of the graph of the backlash model 121 of FIG. 4.

$$\hat{\theta}_{s,i}^{act} = \hat{\theta}_{s,i-1}^{act} + m\dot{\theta}_m^{act} dt \qquad (1)$$

where $\hat{\theta}_{s,i}^{act}$ denotes the bending angle of the scope 150 when time is $i$, $\hat{\theta}_{s,i-1}^{act}$ denotes the bending angle of the scope 150 when time is $i$-1, $m$ denotes the slope of the straight line of the graph, and $\dot{\theta}_m^{act}$ denotes the moving speed of the motor. The slope $m$ of the graph may be determined according to the angle of the motor.

That is, the control unit 120 may model backlash, and may then estimate the pose of the scope 150 based on the state of the motor that provides power to the scope 150. In a situation where the actual endoscope device 100 is operating, the state of the motor (e.g., the pose information of the motor) may be input to the backlash model 121 in real time according to the movement of the endoscopic scope 150. Since the scope 150 does not move in the backlash section, an endoscopic operator may perform an action that excessively moves the endoscopic scope 150 in the backlash section. In this case, when the drive unit 130 generates force equivalent to the input amount of manipulation, the movement of the scope 150 may deviate from a predictable range. The endoscope device 100 according to the present disclosure may precisely perform the manipulation of the scope 150 desired by an endoscopic operator by estimating the pose of the scope 150 using the backlash model 121 in which nonlinearity is taken into consideration.

Referring to FIGS. 2 and 4 together, the control unit 120 may utilize an artificial neural network model 122 configured to estimate the pose of the scope 150.

The control unit 120 may train the artificial neural network model 122 to infer the pose of the scope 150 by using the pose information of the drive unit 130 configured to control the movement of the scope 150 as training data in step S210. Meanwhile, although a case where the artificial neural network model 122 is trained inside the endoscope device 100 will be described as an example, the artificial neural network model 122 may be trained in an external server.

The artificial neural network model 122 may be trained to output the pose information of the scope 150 through supervised learning by using the pose information of the scope 150, the pose information of the motor, and the direction information of the motor as training data. The training data may be prepared by setting a preset time section as a unit. Although the artificial neural network model 122 may be implemented as a Multi Layer Perceptron (MLP), Vanilla RNN, LSTM, or Piecewise regression with decision tree, it is not limited thereto.

In this case, the output pose information of the scope 150 may vary according to the flow of time of training data in which the training data was obtained. That is, instead of receiving information about the backlash of the motor as training data, the artificial neural network model 122 may construct training data as time-series data according to the flow of time and use the training data. As a result, the artificial neural network model 122 may perform training by taking into consideration the backlash section of the scope 150. To this end, the artificial neural network model 122 may be implemented as a model such as RNN or LSTM that is trained by taking into consideration the characteristics of time-series data.

The control unit 120 may estimate the pose of the scope 150 based on the pose information of the drive unit 130 by using the trained artificial neural network model 122 in step S220. The control unit 120 may input the pose information, obtained from the drive unit 130, to the artificial neural network model 122, and may receive the pose information of the scope 150 from the artificial neural network model 122.

After step S130 or S140 of FIG. 3 and after step S220 of FIG. 5, the control unit 120 may control the scope 150 based on the estimated pose information of the scope 150. For example, based on the estimated pose information of the scope 150, there may be performed the computation of the force to be generated by the drive unit 130 in order to provide power to the scope 150 or to compensate the generated power.

The description of the present disclosure described above is intended for illustrative purposes, and those of ordinary skill in the art to which the present disclosure pertains can appreciate that the present disclosure may be easily modified into other specific forms without changing the technical spirit or essential features of the present disclosure. Therefore, it should be understood that the embodiments described above are illustrative and not restrictive in all respects. For example, each component described as being in a single form may be implemented in a distributed form. In the same manner, components described as being in a distributed form may be implemented in a combined form.

The scope of the present disclosure is defined by the following claims rather than the above detailed description, and all changes or modifications derived from the meanings and scope of the claims and their equivalent concepts should be interpreted as being encompassed within the scope of the present disclosure.

**Claims**

1. A method of estimating a pose of an endoscopic scope, the method being performed by a computing device including at least one processor, the method comprising:
estimating a pose of an endoscopic scope based on pose information of a drive unit configured to control movement of the scope by using a backlash model in which control parameters have been identified for backlash that occurs in the scope.

2. The method of claim 1, wherein the backlash model is determined according to an angle range of the scope.

3. The method of claim 2, wherein the backlash model is set such that an amount of backlash is constant when an angle of the scope is within a first range.

4. The method of claim 2, wherein the backlash model is set such that an amount of backlash increases when an angle of the scope is within a second range.

5. The method of claim 1, wherein estimating the pose of the scope comprises determining whether the scope is in a backlash state based on the backlash model and a state of the drive unit.

6. The method of claim 5, wherein estimating the pose of the scope comprises estimating that the pose of the scope is constant in a backlash state.

7. The method of claim 5, wherein estimating the pose of the scope comprises estimating a bending angle of the scope based on an angle of the motor when the scope is not in a backlash state.

8. A method of estimating a pose of an endoscopic scope, the method being performed by a computing device including at least one processor, the method comprising:
estimating a pose of an endoscopic scope based on pose information of a drive unit configured to control movement of the scope by using an artificial neural network model that is trained to infer a pose of the scope by using the pose information of the drive unit as training data.

9. The method of claim 8, wherein the pose information of the drive unit includes pose information of a motor configured to provide power to the scope and direction information of the motor.

10. A computing device for estimating a pose of an endoscopic scope, the computing device comprising:

   a processor including at least one core; and
   memory including program codes executable on the processor;
   wherein the processor estimates a pose of an endoscopic scope based on pose information of a drive unit configured to control movement of the scope by using a backlash model in which control parameters have been identified for backlash that occurs in the scope.

11. A computing device for estimating a pose of an endoscopic scope, the computing device comprising:

   a processor including at least one core; and
   memory including program codes executable on the processor;
   wherein the processor estimates a pose of an endoscopic scope based on pose information of a drive unit configured to control movement of the scope by using an artificial neural network model that is trained to infer a pose of the scope by using the pose information of the drive unit as training data.

FIG. 1

FIG. 2

FIG. 3

Start

Estimate Pose of Endoscopic Scope Based on
Pose Information of Drive Unit by Using Backlash Model
in Which Control Parameters Have Been Identified
for Backlash that Occurs in Scope — S110

S120

Determine Whether Scope
Is in Backlash State Based on Backlash Model
and State of Drive Unit

S130

Estimate that Pose
of Scope Is Constant

S140

Estimate Bending Angle
of Scope Based on Angle
of Motor

End

FIG. 4

FIG. 5

```
                    ( Start )
                        │
                        ▼
┌─────────────────────────────────────────────┐
│         Train Artificial Neural Network Model │
│ to Infer Pose of Scope by Using Pose Information │ ─── S210
│    of Drive Unit Configured to Control Movement  │
│            of Scope as Training Data            │
└─────────────────────────────────────────────┘
                        │
                        ▼
┌─────────────────────────────────────────────┐
│           Estimate Pose of Scope              │
│    Based on Pose Information of Drive Unit     │ ─── S220
│  by Using Trained Artificial Neural Network Model │
└─────────────────────────────────────────────┘
                        │
                        ▼
                    (  End  )
```

Europäisches
Patentamt

European
Patent Office

Office européen
des brevets

# EUROPEAN SEARCH REPORT

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | HAO JIANXIONG ET AL: "Two-Dimensional Shape and Distal Force Estimation for the Continuum Robot Based on Learning From the Proximal Sensors", IEEE SENSORS JOURNAL, IEEE, USA, vol. 23, no. 10, 30 March 2023 (2023-03-30), pages 10836-10846, XP011940571, ISSN: 1530-437X, DOI: 10.1109/JSEN.2023.3262019 [retrieved on 2023-03-30] * page 10836 - page 10846; figures 1-4, 7 * | 1-11 | INV. A61B1/00 A61B1/005 |
| X | US 2013/261392 A1 (YAMAMOTO EIJI [JP] ET AL) 3 October 2013 (2013-10-03) * paragraphs [0091] - [0094]; figure 5 * | 1-7,10 | |

TECHNICAL FIELDS SEARCHED (IPC)

A61B

The present search report has been drawn up for all claims

| Place of search | Date of completion of the search | Examiner |
|---|---|---|
| Munich | 9 April 2025 | Höß, Theresa |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

## ANNEX TO THE EUROPEAN SEARCH REPORT
## ON EUROPEAN PATENT APPLICATION NO.

EP 24 21 4418

This annex lists the patent family members relating to the patent documents cited in the above-mentioned European search report.
The members are as contained in the European Patent Office EDP file on
The European Patent Office is in no way liable for these particulars which are merely given for the purpose of information.

09-04-2025

| Patent document cited in search report | Publication date | Patent family member(s) | Publication date |
|---|---|---|---|
| US 2013261392 A1 | 03-10-2013 | CN 103228193 A | 31-07-2013 |
| | | EP 2647327 A1 | 09-10-2013 |
| | | JP 5766940 B2 | 19-08-2015 |
| | | JP 2012115521 A | 21-06-2012 |
| | | US 2013261392 A1 | 03-10-2013 |
| | | WO 2012074016 A1 | 07-06-2012 |

EPO FORM P0459

For more details about this annex : see Official Journal of the European Patent Office, No. 12/82

# EP 4 559 376 A1

## REFERENCES CITED IN THE DESCRIPTION

**Patent documents cited in the description**

- KR 1020230162122